# EUROPEAN PATENT SPECIFICATION

(11) **EP 1 487 513 B1**
(45) Date of publication and mention of the grant of the patent: **13.06.2012**
(21) Application number: 03745147.3
(22) Date of filing: 19.03.2003
(51) Int. Cl.: A61L 31/04, A61L 31/10

(54) **BIODEGRADABLE HYDROPHOBIC POLYMER FOR STENTS**
BIOABBAUBARES HYDROPHOBES POLYMER FÜR STENTS
POLYMERE HYDROPHOBE BIODEGRADABLE POUR ENDOPROTHESE

(30) Priority: 20.03.2002 US 104772
(43) Date of publication of application: 22.12.2004
(73) Proprietor: Abbott Cardiovascular Systems Inc., Santa Clara, CA 95054 (US)
(72) Inventor: HOSSAINY, Syed, F., A., Fremont, CA 94555 (US); DUTTA, Debashis, Santa Clara, CA 95050 (US)
(74) Representative: Keen, Celia Mary
(86) International application number: PCT/US2003/008474
(87) International publication number: WO 2003/080147

(56) References cited:
- WO-A-00/44309
- WO-A-00/74744
- WO-A-99/34750
- US-A- 6 071 266
- JUSTIN HANES, MSATOSHI CHIBA AND ROBERT LANGER: "Synthesis and Characterization of Degradable Anhydride-co-imide Terpolymers containing Trimellitylimido-L-tyrosine: Novel Polymers for Drug Delivery P", MACROMOLECULES, vol. 29, 1996, pages 5279-5287,

## Description

### 1. Field of the invention

This invention relates to implantable medical devices such as stents. More particularly, the invention relates to materials from which a stent can be made or with which a stent can be coated.

### 2. Description of related art

In the treatment of vascular disease, such as arteriosclerosis, intracoronary stent placement is a common adjunct therapy with balloon angioplasty. Stents eliminate vasospasm, tack dissections to the vessel wall, reduce negative remodeling, and maintain the patency of the vessel. To the extent that the mechanical functionality of stents has been optimized in recent years, stents can cause some undesirable effects. For example, the continued exposure of a stent to blood can lead to thrombus formation, and the presence of a stent in a blood vessel can over time cause the blood vessel wall to weaken, which creates the potential for an arterial rupture or the formation of aneurisms. A stent can also become so overgrown by tissue after its implantation that its usefulness may be substantially decreased while its continued presence may cause a variety of problems or complications. It is therefore desirable for the stent to be biodegradable or bioabsorbable so as to diminish the adverse risks that would otherwise be associated with the stent's continued presence once its usefulness at the treatment site has ceased or has at least become substantially diminished.

To obviate such complications, stents can be fabricated of materials that are biodegradable or bioabsorbable. It is necessary to select a material that is both biodegradable and biocompatible, and, additionally, has suitable physical and mechanical properties necessary to properly serve its function as a stent. Such physical properties include, among others, sufficient strength to support the loads to which the stent is subjected, the radial flexibility necessary for the stent to undergo expansion, and longitudinal flexibility to allow the stent to be advanced through a contorted vasculature and to adapt to a non-linear deployment site.

The necessary characteristics described above have been achieved with the use of polymers such as polylactic acid, copolymers of lactic acid and glycolic acid, and polycaprolactone. However, previously known biodegradable or bioabsorbable stents typically exhibit bulk erosion and are as a consequence prone to breaking-up into large particles as the matrix breaks down. The bulk erosion of such materials is likely to additionally increase if they are also used to make stent coatings, for the local delivery of drugs, thus causing the coating to flake off or otherwise become detached. Should large particles become dislodged before becoming completely degraded, they could be washed downstream and cause emboli.

Hydrophobic polymers with water-labile linkages can serve as biodegradable or bioabsorbable materials having satisfactory resistance to bulk erosion. However, such polymers may have only a limited strength and processing capabilities. Consequently, their use has been restricted to passive devices that neither perform a structural function nor are subject to stress or distortion. The use of such polymers in stent applications has been precluded as they are unable to support a lumen wall or remain attached to a stent as it undergoes deformation during its expansion.

WO00/74744 and WO00/44309 separately describe a stent made of a biologically degradable or absorbable hydrophobic polymer such as poly-L-lactic acid, poly-L-glycolic acid.

WO99/34750 refers to a stent made of a biologically degradable or absorbable hydrophobic polymer, e.g. poly-L-lactic acids.

US6071266 describes a stent made of a biologically degradable or absorbable hydrophobic polymer, e.g. polyurethanes.

In view of the foregoing, a biodegradable stent is therefore needed that is capable of providing the necessary structural support to a body lumen and then gradually and completely degrading or absorbing in a manner that precludes break-up into large particles. Similarly, a biodegradable coating is needed that is not prone to flaking or breaking-up into particles which may cause embolic complications.

### SUMMARY OF THE INVENTION

In accordance to one embodiment of the invention, a stent made of, or comprising a coating which includes, a biologically degradable or absorbable hydrophobic polymer is provided. The polymer is an anhydride-co-imide terpolymer comprising fragments having water-labile imide bonds, wherein the polymer contains the following three kinds of units: (a) units derived from trimellitylimido-L-tyrosine (TMIT); (b) units derived from sebacic acid (SBA); and (c) units derived from 1,3-bis(para-carboxyphenoxy) propane (PCPP).

In one embodiment, the stent is coated with the above-described material and the therapeutic substance is incorporated into the coating. In another embodiment, a biologically active substance is chemically bonded to the backbone of the polymer or incorporated into the backbone of the polymer.

### DETAILED DESCRIPTION

The polymeric materials used in the embodiments of the present invention to fabricate a stent and/or a coating for a stent are selected based on strength characteristics as well as the materials' tendency to gradually erode from the surface rather than being subject to bulk erosion. Drugs or other pharmacologically or therapeutically active agents that can be incorporated within such material can be gradually released as the polymer degrades. Materials that exhibit the desired surface eroding characteristics without being subject to bulk erosion include polymers having the degradation rate higher than the rate of water penetration into the interior of the polymeric mass. Such polymers are hydrophobic as a whole but at the same time have water-labile linkages interconnecting the monomeric units comprising the polymers. As a result, the stent and/or the stent coating gradually degrades from the outermost surface inwardly, due to the slow hydrolysis of the polymers at the polymers' vulnerable water-labile linkages. This process of degradation of the polymers takes place without a great risk of large particles getting dislodged.

Materials employed to fabricate the stents and/or the stent coatings of the present invention include hydrophobic polymers which have water-labile linkages interconnecting the monomeric units. The selected polymers can further include ester or imide fragments. The overall hydrophobic nature of the polymer precludes the incursion of water into the polymer's interior while the water-labile bonds that are exposed on the polymer's surface nonetheless cause the polymer to degrade. Degradation thereby progresses from the material's outermost surface inwardly to yield a uniform degradation rate and to preclude bulk erosion.

Using polymers having the imide or ester bonds to fabricate the stents of the present invention serves to impart sufficient strength to the material to enable the polymer to provide the support that is required of a stent. Alternatively, if the material is used to make the stent coating, the incorporation of the imide or ester fragments impart sufficient strength to the material to prevent it from flaking off or otherwise becoming detached as the underlying stent undergoes distortion cause by the radial expansion of the stent.

One of the ways to control the stent's and/or the stent coating's ultimate performance characteristics is by varying the chemical variables of the material used to fabricate the stent and/or the stent coating. For example, the number of imide or ester bonds that are incorporated in the polymer material not only affects the ultimate strength and flexibility of the stent and/or the stent coating, but also has an effect on the rate at which the material degrades when subjected to blood flow. An increased bond content enhances strength, decreases flexibility and increases degradation time.

Polymers that satisfy the above-described requirements include polyanhydrides and polyesters or polyorthoesters. The present invention employs a polyanhydride polymer which is suitable for use in the construction of a stent and/or fabrication of the stent coating, which is an anhydride-co-imide terpolymer containing the following three kinds of units:
(a) units derived from trimellitylimido-L-tyrosine (TMIT), a product of acylation, via imide nitrogen, of L-tyrosine by imide of trimellitic anhydride having the formula (I) 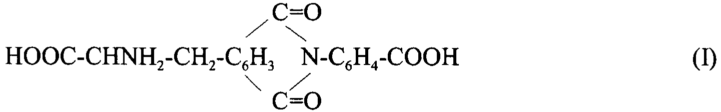
(b) units derived from sebacic acid (SBA) (which is also known as decainedioic acid) having the formula HOOC-(CH₂)₈-COOH ; and
(c) units derived from 1, 3-bis (para-carboxyphenoxy)propane (PCPP) having the formula HOOC-*p*-C₆H₄-(CH₂)3-*p*-C₆H₄-COOH.

A general formula of this terpolymer, poly[trimellitylimido-L-tyrosine-co-sebacic acid-co-1,3-bis(*para*-carboxyphenoxy)propane], p(TMIT-SBA-PCPP), is shown below as formula (II).

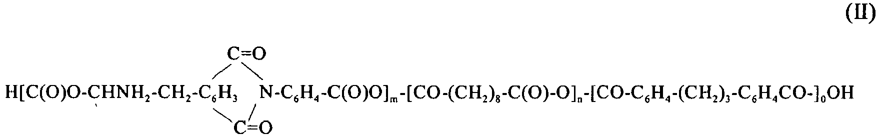

In the terpolymer of formula (II) the"m"units are derived from TMIT, the "n" units from SBA, and the "o" units from PCPP. The "m" units provide the terpolymer of formula (I) with cyclic fragments having water-labile imide bonds-N[(C=O)]₂. These fragments provide the terpolymer with acidic properties because the imide anion is well stabilized via the resonance structures. For p(TMIT-SBA-PCPP) to be effective to provide sufficient strength for a stent application, a content of the imide-derived "m" units of between 20% and 40% (mass) can be used.

Since body fluids, such as blood, are slightly alkaline (pH ∼ 7. 3), the imide bonds will be hydrolyzed via S_{N}2 mechanism upon contact with blood. The rate of such nucleophilic reaction of base-catalyzed hydrolysis is low. Hence, the stent and/or the stent coating made of the terpolymer of formula (II) will be slowly degraded when exposed to blood.

Another example of a suitable polyanhydride includes a copolymer of 1,6-bis(*para*-carboxyphenoxy)hexane (PCPX) having the formula

HOOC-*p*-C₆H₄-(CH₂)₆-*p*-C₆H₄-COOH

and di-*ortho*-carboxyphenoxy sebacate anhydride (OCPSA) having the formula 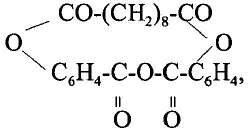 where the anhydride substituents of the sebacate portion of the copolymer are in the *ortho*-positions of the correspondent benzene rings. A general formula of this copolymer, p(PCPX-OCPSA) is shown below as formula (III).

HO[-OC-*p*-C₆H₄-(CH₂)₆-*p*-C₆H₄-CO]ₚ-[OC(O)-C₆H₄-OCO-(CH₂)₈-C(O)O-C₆H₄CO-]_{q} (III)

In the copolymer of formula (III), the "p" units are derived from PCPX, and the "q" units are derived from OCPSA. This copolymer is suitable for making stents and/or coatings for such stents because, as a result of biological degradation while in contact with body fluids, p(PCPX-OCPSA) releases salicylic (2-hydroxybenzoic) acid (SA). SA having the formula HO-*ortho*-C₆H₄-COOH is an antiplatelet agent.

Another example of a suitable polyanhydride includes poly (maleic acid-co-sebacic acid), p(MA-SBA), synthesized from maleic acid (MA) having the formula HOOC-CH=CH-COOH or anhydride thereof, and sebacic acid or anhydride thereof.

Another example of a suitable polyanhydride includes terpolymers including the units derived from PCPP, SBA and SA. This terpolymer, poly[1,3-bis(*para*-carboxyphenoxy) propane-co-sebacic acid-co-salicylic acid], p(PCPP-SBA-SA), is similar to p(TMIT-SBA-PCPP) shown by formula II, except that trimellitylimido-L-tyrosine units are substituted with salicylic acid units. Thus, in p(PCPP-SBA-SA), units derived from salicylic acid constitute a part of the backbone of the copolymer. Consequently, SA is released upon biological degradation of p(PCPP-SBA-SA).

Yet other examples of a suitable polyanhydride include poly(L-lactic acid), pLLA, poly(DL-lactic acid), pDLLA, and products of polycondensation of L- or DL-lactic acid with other multifunctional acids.

Examples of such products of polycondensation include poly(L-lactic acid-co-L-aspartic acid), p(LLA-LAspA), or poly(DL-lactic acid-co-L-aspartic acid), p(DLLA-LAspA). p(LLA-LAspA) or p(DLLA-LAspA) can be synthesized from either L- or DL- optical isomer of lactic acid, CH₃-CH(OH) -COOH, (LLA and DLLA, respectively) and L-aspartic acid (LAspA), also known as aminosuccinic acid, or 1-amino-1,2-dicarboxyethane, which is an amino acid having the formula HOOC-CH₂-CH(NH₂)-COOH.

Polyesters are another class of biologically degradable polymers suitable for making stents and/or stent coatings. Polyesters contain fragments with ester bonds which are water-labile bonds, similar to that of imide bonds. When contact with slightly alkaline blood these bonds are subject to catalyzed hydrolysis, thus ensuring biological degradability of the polyester. Representative examples of polyester polymers suitable for use in the construction of a stent and/or fabrication of the stent coating include poly(hydroxybutyrate) (PHB), poly(hydroxyvalerate) (PHV), and poly(hydroxybutyrate-valerate) (PHBV), which is copolymer of hydroxybutyrate and hydroxyvalerate, as well as poly(4-hydroxy-L-proline ester), p(HOXPE) which is a product of self-polycondensation of 4-hydroxy-L-proline (HOXP). HOXP having an imide bond provides p(HOXPE) with fragments comprising both ester bonds and imide bonds. As a result, the biological degradation of p(HOXPE) is facilitated.

Other examples of polyesters suitable for making a stent and/or a stent coating include, poly(1,10-decanediol-1,10-decanediol dilactide), p(DCD-LLA) or p(DCD-DLLA), which is a product of polycondensation of a relatively long-chained glycol, 1,10-decanediol (DCD) and either L- or DL-lactic acid, LLA or DLLA, respectively.

Another example of a suitable polyorthoester that can be used to make stents and/or stent coatings is a block-copolymer of either LLA or DLLA with ethylene glycol (EG). Such block-copolymer, poly[L-(or DL-) lactic acid-co-ethylene glycol], p(LLA-EG) or p(DLLA-EG), comprising units derived from EG and from lactic acid (either L- or DL- optical isomer, as the case may be), can be synthesized by polymerization of LLA or DLLA induced by the poly(ethylene glycol) (PEG) macroinitiator.

While in contact with the body fluids, p(LLA-EG) or p(DLLA-EG) degrades to yield monomeric lactic acid and PEG. Both of these products are biologically compatible, and PEG also has an additional advantage of being biologically active, reducing smooth muscle cells proliferation at the lesion site and thus capable of inhibiting restenosis.

Another example of the PEG-containing polyester, suitable for making a stent and/or a stent coating in accordance with the present invention includes a block-copolymer of EG with butyleneterephthalate (BT). This block-copolymer can be obtained, for example, by trans-esterification of dibutylterephthalate with PEG.

Yet another example of a polyester suitable making a stent and/or a stent coating in accordance with the present invention includes poly(1,2,6-hexanetriol-trimethylorthoacetate), p(HTOL-TMAC), a product of polycondensation of a trifunctional alcohol 1,2,6-hexanetriol (HTOL) and trimethylacetic acid.

For any of the polyester polymers described above, a content of ester-derived units of between 20% and 40% (mass) is effective to provide sufficient strength for a stent application.

Table 1 presents the summary of these polymers and also shows the monomers used to synthesize the polymers.

**Table 1. Polymers Usable for Fabrication of Biodegradable Stents And/Or Stent Coatings**

| No. | Polymer and abbreviation | Monomer 1 and abbreviation | Monomer 2 and abbreviation | Monomer 3 and abbreviation |
|---|---|---|---|---|
| 1 | Poly[trimellitylimido-L-tyrosine-co-sebacic acid-co-1,3-bis(*para*-carboxyphenoxy)propane], p(TMIT-SBA-PCPP) | Trimellitylimido-L-tyrosine (TMIT) | Sebacic acid (SBA) | 1,3-bis (*para*-carboxyphenoxy) propane (PCPP) |
| 2 | Poly[1,6-bis(*para*-carboxyphenoxy)hexane-co-di-o carboxyphenoxy sebacate anhydride], p(PCPX-OCPSA) | 1,6-bis (*para*-carboxyphenoxy) hexane (PCPX) | di-*ortho*-carboxyphenoxy sebacate anhydride (OCPSA) | None |
| 3 | Poly[1,3-bis(*para*-carboxyphenoxy) propane-co-sal acid-co- sebacic acid], p(PCPP-SBA-SA) | 1,3-bis(*para*-carboxyphenoxy) propane (PCPP) | Sebacic acid or anhydride (SBA) | Salicylic acid (SA) |
| 4 | Poly(maleic acid-co-sebacic acid), p(MA-SBA) | Maleic acid (MA) | Sebacic acid or anhydride (SBA) | None |
| 5 | Poly(L-lactic acid-co-L-aspartic acid), p(LLA-LAspA) | L-lactic acid (LLA) | L-aspartic acid (LAspA) | None |
| 6 | Poly(DL-lactic acid-co-L-aspartic acid), p(DLLA-LAspA) | DL-lactic acid (DLLA) | L-aspartic acid (LAspA) | None |
| 7 | Poly(L-lactic acid), pLLA | L-lactic acid (LLA) | None | None |
| 8 | Poly(DL-lactic acid), pDLLA | DL-lactic acid (DLLA) | None | None |
| 9 | Poly(L-lactic acid-co-ethylene glycol), p(LLA-EG) | L-lactic acid (LLA) | Ethylene glycol (EG) | None |
| 10 | Poly(DL-lactic acid-co-ethylene glycol), p(DLLA-EG) | DL-lactic acid (DLLA) | Ethylene glycol (EG) | None |
| 11 | Poly(ethylene glycol-co-butylene terephthalate), p(EG-BT) | Ethylene glycol (EG) | Butylene terephthalate) (BT) | None |
| 12 | Poly(4-hydroxy-L-proline ester), p(HOXPE) | 4-hydroxy-L-proline (HOXP) | None | None |
| 13 | Poly(1,10-decanediol-1,10-decanediol dilactide), p(DCD-LLA) | 1,10-decanediol (DCD) | L-lactic acid (LLA) | None |
| 14 | Poly(1,10-decanediol-1,10-decanedioldilactide), p(DCD-DLLA) | 1,10-decanediol (DCD) | DL-lactic acid (DLLA) | None |
| 15 | Poly(1,2,6-hexanetriol-trimethylorthoacetate), p(HTOL-TMAC) | 1,2,6-hexanetriol (HTOL) | Trimethylacetic acid(anhydride) (TMAC) | None |
| 16 | Poly(hydroxybutyrate) (PHB) | Hydroxybutyrate (HB) | None | None |
| 17 | Poly(hydroxyvalerate) (PHV) | Hydroxyvalerate (HV) | None | None |
| 18 | Poly(hydroxy-butyrate-valerate) (PHBV) | Hydroxybutyrate (HB) | Hydroxyvalerate (HV) | N/A |

A stent of the present invention can be formed by first causing the appropriate reagents to react to form the desired polyanhydride or polyester composition. During copolymer synthesis, the imide content of such composition is increased by incorporating higher imide-containing monomers like trimellitylimido-L-tyrosine. Increasing imide content results in higher material strength. The ester content of such composition is increased by incorporating higher ester containing monomers such as L-proline ester or trimethyl orthoacetate. Flexibility of polyanhydrides like p(MA-SBA) can be increased by increasing the percentage of maleic acid dimer (MAD) during polymer synthesis.

Therapeutically active agents can be optionally added to the reagents so as to incorporate such agents throughout the polymer to thereby provide for the gradual dispensation of the agent over the service life of the matrix. The blending may be accomplished either in solution or in a melt state. Alternatively, some active agents may be infused throughout the polymer after polymerization is completed. Some active agents can be chemically incorporated into the backbone of the polymer, or can be chemically bonded to the polymer backbone as a pendant group. One example of such an active agent is salicylic acid. Other examples of active agents that can be chemically incorporated into the polymer include nitric oxide, PEG, heparin and its derivatives, and hyaluronic acid.

The therapeutically active agent can include any substance capable of exerting a therapeutic or prophylactic effect in the practice of the present invention. The agent may include small or large molecule drugs, peptides, proteins, oligonucleotides, or DNA. Examples include antiproliferative substances such as actinomycin D, or derivatives and analogs thereof. Synonyms of actinomycin D include dactinomycin, actinomycin IV, actinomycin I₁, actinomycin X₁, and actinomycin C₁. The active agent can also fall under the genus of antineoplastic, anti-inflammatory, antiplatelet, anticoagulant, antifibrin, antithrombin, antimitotic, antibiotic, antiallergic and antioxidant substances. Examples of such antineoplastics and/or antimitotics include paclitaxel, docetaxel, methotrexate, azathioprine, vincristine, vinblastine, fluorouracil, doxorubicin hydrochloride, and mitomycin. Examples of such antiplatelets, anticoagulants, antifibrin, and antithrombins include sodium heparin, low molecular weight heparins, heparinoids, heparin derivatives having hydrophobic counter ions, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonism antibody, recombinant hirudin, and thrombin. Examples of such cytostatic or antiproliferative agents include angiopeptin, angiotensin converting enzyme inhibitors such as captopril, cilazapril or lisinopril, calcium channel blockers (such as nifedipine), colchicine, fibroblast growth factor (FGF) antagonists, fish oil (ω-3- fatty acid), histamine antagonists, lovastatin (an inhibitor of HMG-CoA reductase, a cholesterol lowering drug), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), and nitric oxide. An example of an antiallergic agent is permirolast potassium. Other therapeutic substances or agents which may be appropriate include alpha-interferon, genetically engineered epithelial cells, rapamycin and its derivatives (one example of which is everolimus available from Novartis Corp.), and dexamethasone.

Although the embodiments of the present invention have been described with reference to a stent, such as a balloon expandable or self-expandable stent, other implantable medical devices can also be made from or coated with the polymers. Examples of the implantable medical device include stent-grafts, grafts (e.g., aortic grafts), artificial heart valves, cerebrospinal fluid shunts, pacemaker electrodes, coronary shunts and endocardial leads (e.g., FINELINE and ENDOTAK, available from Guidant Corporation). The underlying structure of the device can be of virtually any design.

The stent may be formed by any of a number of well known methods including the extrusion of the polymer into the shape of a tube. Pre-selected patterns of voids can then be formed into the tube in order to define a plurality of spines or struts that impart a degree of flexibility and expandability to the tube. Alternatively, the drug loaded polymer may be applied to the selected surfaces of a stent made of, for example, stainless steel. The stent can be, for example, immersed in the molten polymer or sprayed with a liquid form of the polymer. In yet another exemplary embodiment, the polymer may be extruded in the form of a tube which is then co-drawn with a tube of stainless steel, or other suitable metallic materials or alloys. By co-drawing two tubes of the polymer with the metal tube, one positioned about the exterior of the metal tube and another positioned within the metal tube, a tube having multi-layered walls is formed. Subsequent perforation of the tube walls to define a pre-selected pattern of spines or struts imparts the desired flexibility and expandability to the tube to create a stent.

The polymer listed in Table 1 can be further blended or coated with one or a plurality of other polymers, referred to herein as "alternative polymers." One example of an alternative polymer is poly(ethylene-co-vinyl alcohol), also known under the trade name EVAL and distributed commercially by Aldrich Chemical Company of Milwaukee, Wisconsin. EVAL is also manufactured by EVAL Company of America, Lisle, Illinois. EVAL is a product of hydrolysis of ethylene-vinyl acetate copolymers. EVAL may also be a terpolymer and may include up to 5% (molar) of units derived from styrene, propylene and other suitable unsaturated monomers.

Other examples of alternative polymers include poly (hydroxyvalerate), polycaprolactone, poly(lactide-co-glycolide), poly(hydroxybutyrate), poly(hydroxybutyrate-co-valerate), polydioxanone, poly(glycolic acid), polyphosphoester, polyphosphoester urethane, poly(amino acids), cyanoacrylates, poly(iminocarbonate), copoly(ether-esters) (e. g. PEO/PLA), polyalkylene oxalates, polyphosphazenes, polyurethanes, silicones, polyolefins, polyisobutylene and ethylene-alphaolefin copolymers, acrylic polymers and copolymers, vinyl halide polymers and copolymers, such as polyvinyl chloride, polyvinyl ethers, such as polyvinyl methyl ether, polyvinylidene halides, such as polyvinylidene fluoride and polyvinylidene chloride, polyacrylonitrile, polyvinyl ketones, polyvinyl aromatics, such as polystyrene, polyvinyl esters, such as polyvinyl acetate, copolymers of vinyl monomers with each other and olefins, such as ethylene-methyl methacrylate copolymers, acrylonitrile-styrene copolymers, ABS resins, and ethylene-vinyl acetate copolymers, polyamides, such as Nylon 66 and polycaprolactam, alkyd resins, polycarbonates, polyoxymethylenes, polyimides, polyethers, epoxy resins, polyurethanes, rayon, rayon-triacetate, cellulose, cellulose acetate, cellulose butyrate, cellulose acetate butyrate, cellophane, cellulose nitrate, cellulose propionate, cellulose ethers, carboxymethyl cellulose, and biomolecules, such as fibrin, fibrinogen, cellulose, starch, collagen and hyaluronic acid.

The following examples are provided for illustrative purposes.

### Reference Example 1

A first composition can be prepared by mixing the following components:
(a) between about 0.1 mass % and about 15 mass %, for example, about 1.0 mass % of p(LLA-EG);
(b) between about 0.1 mass % and about 10.0 mass %, for example, about 2.0 mass % of p(DLLA);
(c) between about 0.05 mass % and about 2.0 mass %, for example, about 1.0 mass % of a biologically active substance such as rapamycin, or a derivative or analog thereof; and
(d) the balance, dioxane solvent.

The first composition is applied onto the stent and dried to form a drug-polymer layer. The composition is applied onto the stent by any conventional method, for example, by spraying or dipping. A primer layer (e.g., the above formulation without the therapeutically active substance) can be optionally applied on the surface of the bare stent prior to the application of the drug-polymer layer.

For a stent having a length of 13 mm and diameter of 3 mm, the total amount of solids of the matrix layer can be about 300 micrograms (corresponding to the thickness of between about 15 and 20 microns). "Solids" means the amount of the dry residue deposited on the stent after all volatile organic compounds (e.g., the solvent) have been removed.

A second composition can be prepared by mixing the following components:
(a) between about 0.1 mass % and about 15 mass %, for example, about 1.0 mass % of p(LLA-EG);
(b) between about 0.1 mass % and about 10.0 mass %, for example, about 2.0 mass % of p(DLLA); and
(c) the balance, dioxane solvent.

The second composition is applied onto the dried drug-polymer layer and dried, to form an optional topcoat layer. The topcoat layer can be applied by any conventional method and can have, for example, a total solids weight of about 200 µg.

A third composition can be prepared by mixing the following components:
(a) between about 0.1 mass % and about 15 mass %, for example, about 1.125 mass % of p(LLA-EG);
(b) between about 0.1 mass % and about 10.0 mass %, for example, about 0.75 mass % of p(DLLA); and
(c) the balance, dioxane solvent.

The third composition is applied onto the topcoat layer and dried, to form an optional finishing coat layer. The finishing coat layer can be applied by any conventional method and can have, for example, a total solids weight of about 150 µg.

### Reference Example 2

A first composition can be prepared by mixing the following components:
(a) between about 0.1 mass % and about 15 mass %, for example, about 1.0 mass % of p(LLA-EG);
(b) between about 0.1 mass % and about 10.0 mass %, for example, about 2.0 mass % of p(DLLA);
(c) between about 0.05 mass % and about 2.0 mass %, for example, about 1.0 mass % of estradiol; and
(d) the balance, dioxane solvent.

The first composition is applied onto a stent to form a drug-polymer layer with about 300 µg of total solids.

A second composition can be prepared by mixing the following components:
(a) between about 0.1 mass % and about 15 mass %, for example, about 1.0 mass % of p(LLA-EG);
(b) between about 0.1 mass % and about 10.0 mass %, for example, about 2.0 mass % of p(DLLA); and
(c) the balance, dioxane solvent.

The second composition is applied onto the dried drug-polymer layer and dried to form an optional topcoat layer. The topcoat layer can have, for example, a total solids weight of about 200 µg.

A third composition can be prepared by mixing the following components:
(a) between about 0.1 mass % and about 15 mass %, for example, about 1.125 mass % ofp(LLA-EG);
(b) between about 0.1 mass % and about 10.0 mass %, for example, about 0.75 mass % of p(DLLA); and -
(c) the balance, dioxane solvent.

The third composition is applied onto the topcoat layer and dried, to form the optional finishing coat layer. The finishing coat layer can have, for example, a total solids weight of about 150 µg.

### Reference Example 3

A first composition can be prepared by mixing the following components:
(a) between about 0.1 mass % and about 15 mass %, for example, about 2.0 mass % of p(PCPP-SA-SBA);
(b) between about 0.05 mass % and about 2.0 mass %, for example, about 1.0 mass % of estradiol ; and
(c) the balance, a solvent mixture containing about equal mass amounts of dimethylacetamide (DMAC) and tethrahydrofurane (THF).

The first composition is applied onto a stent to form a drug-polymer layer with about 300 µg of total solids.

A second composition can be prepared by mixing the following components:
(a) between about 0.1 mass % and about 15 mass %, for example, about 2.0 mass % of p(PCPP-SA-SBA); and
(c) the balance, a solvent mixture containing about equal mass amounts of DMAC and THF.

The second composition is applied onto the dried drug-polymer layer to form an optional topcoat layer. The topcoat layer can have, for example, a total solids weight of about 200 µg.

The three examples of the formulations above can be summarized as shown in Table 2.

**Table 2. A Summary of the Formulations of Examples 1-3**

| Example | Polymer in matrix layer | Drug in matrix layer | Solids in dry matrix layer, µg | Polymer in topcoat layer | Solids in the dry topcoat layer, µg | Polymer in finishing layer | Solids in the dry finishing layer, µg |
|---|---|---|---|---|---|---|---|
| 1 | p(LLA-EG), 1% and p(DLLA), 2% | a derivative of rapamycin, 1.0% | 300 | p(LLA-EG), 1% and p(DLLA), 2% | 200 | p(LLA-EG), 1.125% and p(DLLA), 0.75% | 150 |
| 2 | p(LLA-EG), 1 % and p(DLLA), 2% | estradiol, 1.0% | 300 | p(LLA-EG), 1% and p(DLLA), 2% | 200 | p(LLA-EG), 1.125% and p(DLLA), 0.75% | 150 |
| 3 | p(PCPP-SA-SBA), 2% | estradiol, 1.0% | 300 | p(PCPP-SA-SBA), 2% | 200 | None | N/A |

## Claims

1. A stent made of, or comprising a coating which includes, a biologically degradable or absorbable hydrophobic polymer, the polymer being an anhydride-co-imide terpolymer comprising fragments having water-labile imide bonds, wherein the polymer contains the following three kinds of units:
(a) units derived from trimellitylimido-L-tyrosine (TMIT);
(b) units derived from sebacic acid (SBA); and
(c) units derived from 1,3-bis(*para*-carboxyphenoxy)propane (PCPP).

2. A stent according to claim 1, wherein the polymer is poly[trimellitylimido-L-tyrosine-co-sebacic acid-co-1,3-bis(*para-*carboxyphenoxy)propane].

3. A stent according to claim 1 or claim 2 wherein the content of the units derived from TMIT in the polymer is between 20% and 40% by mass.

4. A stent according to any one of the preceding claims, further comprising a therapeutic substance contained by the polymer or chemically bonded to the polymer.

5. A stent according to any one of the preceding claims which further comprises a therapeutic substance incorporated into the coating.

6. A stent according to any one of the preceding claims, further comprising a biologically active substance chemically bonded to the backbone of the polymer or incorporated into the backbone of the polymer.

## Patentansprüche

1. Stent, hergestellt aus oder umfassend einer/eine Beschichtung, die ein biologisch abbaubares oder absorbierbares hydrophobes Polymer umfasst, wobei das Polymer ein Anhydrid-co-Imid-Terpolymer ist, das Fragmente, die wasserlabile Imid-Bindungen haben, umfasst, wobei das Polymer die folgenden drei Arten von Einheiten enthält:
(a) Einheiten, die von Trimellitylimido-L-tyrosin (TMIT) abgeleitet sind;
(b) Einheiten, die von Sebacinsäure (SBA) abgeleitet sind, und
(c) Einheiten, die von 1,3-Bis(para-carboxyphenoxy)propan (PCPP) abgeleitet sind.

2. Stent gemäß Anspruch 1, wobei das Polymer Poly[trimellitylimido-L-Tyrosin-co-Sebacinsäure-co-1,3-Bis (para-carboxyphenoxy) propan] ist.

3. Stent gemäß Anspruch 1 oder Anspruch 2, wobei der Gehalt der Einheiten, die von TMIT abgeleitet sind, in dem Polymer zwischen 20 und 40 Massen-% liegt.

4. Stent gemäß einem der vorangehenden Ansprüche, der außerdem eine therapeutische Substanz umfasst, die in dem Polymer enthalten oder chemisch an das Polymer gebunden ist.

5. Stent gemäß einem der vorangehenden Ansprüche, der außerdem eine therapeutische Substanz umfasst, die in die Beschichtung eingearbeitet ist.

6. Stent gemäß einem der vorangehenden Ansprüche, der außerdem eine biologisch aktive Substanz umfasst, die chemisch an die Hauptkette des Polymers gebunden ist oder in die Hauptkette des Polymers eingearbeitet ist.

## Revendications

1. Endoprothèse vasculaire faite en un polymère hydrophobe biologiquement dégradable ou absorbable ou comprenant un revêtement qui renferme un tel polymère, lequel polymère est un terpolymère anhydride-co-imide comportant des fragments dotés d'enchaînements de type imide qui sont labiles à l'eau, et lequel polymère comporte des motifs des trois types suivants :
a) des motifs dérivés de la trimellitylimido-L-tyrosine (TMIT),
b) des motifs dérivés de l'acide sébacique (SBA),
c) et des motifs dérivés du 1,3-bis(para-carboxy-phénoxy)-propane (pCPP).

2. Endoprothèse vasculaire conforme à la revendication 1, dans laquelle le polymère est un poly[trimellitylimido-L-tyrosine-co-(acide sébacique)-co-1,3-bis(para-carboxy-phénoxy)-propane].

3. Endoprothèse vasculaire conforme à la revendication 1 ou 2, dans laquelle la proportion massique des motifs dérivés de la TMIT dans le polymère vaut de 20 % à 40 %.

4. Endoprothèse vasculaire conforme à l'une des revendications précédentes, qui comprend en outre une substance thérapeutique renfermée dans le polymère ou chimiquement liée au polymère.

5. Endoprothèse vasculaire conforme à l'une des revendications précédentes, qui comprend en outre une substance thérapeutique incorporée dans le revêtement.

6. Endoprothèse vasculaire conforme à l'une des revendications précédentes, qui comprend en outre une substance, dotée d'une activité biologique, qui est chimiquement liée au squelette du polymère ou incorporée dans le squelette du polymère.
